# EUROPEAN PATENT SPECIFICATION

(11) **EP 2 069 487 B1**
(45) Date of publication and mention of the grant of the patent: **19.03.2014**
(21) Application number: 07810802.4
(22) Date of filing: 25.07.2007
(51) Int. Cl.: C12N 9/12, C12N 9/00, C12Q 1/68

(54) **ZWITTERIONIC DETERGENTS FOR THE STORAGE AND USE OF DNA POLYMERASES**
ZWITTERIONISCHE REINIGER FÜR DIE LAGERUNG UND VERWENDUNG VON DNA-POLYMERASEN
DETERGENTS ZWITTERIONIQUES POUR LE STOCKAGE ET L'UTILISATION D'ADN POLYMERASES

(30) Priority: 25.07.2006 US 833331 P
(43) Date of publication of application: 17.06.2009
(73) Proprietor: Agilent Technologies, Inc., Santa Clara, CA 95051 (US)
(72) Inventor: HOGREFE, Holly, H., San Diego, CA 92122 (US); FOX, Jeffrey, D., Escondido, CA 92029 (US); BORNS, Michael, Escondido, CA 92026 (US)
(74) Representative: Grünecker, Kinkeldey, Stockmair & Schwanhäusser
(86) International application number: PCT/US2007/016790
(87) International publication number: WO 2008/013885

(56) References cited:
- WO-A1-98/48053
- WO-A1-99/46400
- WO-A1-02/086165
- DE-A1- 19 612 779
- GB-A- 2 420 560
- US-A1- 2002 168 658
- US-B1- 6 242 235
- HENKE WOLFGANG ET AL: "Betaine improves the PCR amplification of GC-rich DNA sequences" NUCLEIC ACIDS RESEARCH, OXFORD UNIVERSITY PRESS, SURREY, GB LNKD- DOI:10.1093/NAR/25.19.3957, vol. 25, no. 19, 1 January 1997 (1997-01-01), pages 3957-3958, XP002171292 ISSN: 0305-1048
- MYTELKA D S ET AL: "ANALYSIS AND SUPPRESSION OF DNA POLYMERASE PAUSES ASSOCIATED WITH A TRINUCLEOTIDE CONSENSUS" NUCLEIC ACIDS RESEARCH, OXFORD UNIVERSITY PRESS, SURREY, GB LNKD- DOI:10.1093/NAR/24.14.2774, vol. 24, no. 14, 1 January 1996 (1996-01-01), pages 2774-2781, XP002070819 ISSN: 0305-1048

## Description

### BACKGROUND

Amplification of nucleic acids involves the thermal cycling of a reaction mixture containing a nucleic acid polymerase to generate an amplified target nucleic acid. An example of this thermal cycling process is that which occurs in Polymerase Chain Reaction (PCR), in which the reaction mixture is subjected to oligonucleotide denaturing, primer annealing, and primer extension reaction temperatures. Thermostable polymerases are generally used to amplify the target nucleic acid sequences in these thermal cycling reactions.

U.S. Patent No. 6,127,155 discloses that the non-ionic detergents NP-40 and Tween stabilize Taq DNA polymerase.

U.S. Publication No. 2003/0017567 discloses a method for performing an amplification reaction utilizing a dye that converts electromagnetic energy into thermal energy to heat the reaction mixture. A zwitterionic surfactant is added to the reaction mixture to reduce interference of the dye with the functioning of the nucleic acid polymerase.

U.S. Publication No. 2002/0168658 discloses the use of zwitterions in combination with a compound that disrupts base paring, e.g., DMSO, to improve the amplification of nucleic acids that are G+C rich.

US 6 242 235 B1 (SHULTZ JOHN W [US] ET AL, 5 June 2001) describes a Taq surfactant solution comprising a purified Taq polymerase, a 10X surfactant buffer including a zwitterionic detergent such as CHAPS or CHAPSO, wherein the detergent enhances the activity of the polymerase.

### SUMMARY

The invention provides compositions, kits and methods that include a polymerase and a zwitterionic detergent or non-detergent surfactant, wherein said zwitterionic detergent is selected from the group consisting of: a combination of n-Decyl-N,N-dimethyl-3-ammonio-1-propanesulfate (Anzergent/Zwittergent 3-10) or n-Dodecyl-N,N-dimethyl-3-ammonio-1-propanesulfonate (Anzergent/Zwittergent 3-12) with CHAPS or CHAPSO, and wherein said non-detergent surfactant is Surfynol 465. Specifically, such compositions, kits and methods are useful in PCR, QPCR, sequencing and mutagenesis. The present invention is based in part on the surprising finding that zwitterionic detergents and non-detergent surfactants increase stability and enhance activity of thermostable polymerases.

In a first aspect, a reaction mixture that includes a purified polymerase, an oligonucleotide probe and a zwitterionic detergent is provided. A detectable label is operatively coupled to the oligonucleotide probe.

In another aspect, a reaction mixture that includes a purified polymerase, a detectable label, and a combination of two or more zwitterionic detergents is provided. In some embodiments, the detectable label is operatively coupled to an oligonucleotide probe.

In another aspect, a reaction mixture having a purified polymerase, a labeled nucleotide, and a zwitterionic detergent or non-detergent surfactant is provided.

In another aspect, the reaction mixture includes a purified polymerase, a fluorescent DNA binding dye, and a zwitterionic detergent or non-detergent surfactant, wherein said fluorescent DNA binding dye produces a detectable signal when bound to DNA.

In yet another aspect, a storage composition having a purified polymerase and a zwitterionic detergent is provided. The storage composition does not contain a detectable label.

In yet another aspect, a storage composition which includes a purified polymerase and a combination of two or more zwitterionic detergents is provided. The storage composition does not contain a detectable label.

In another aspect, a storage composition that includes a purified polymerase, a labeled nucleotide, and a zwitterionic detergent or non-detergent surfactant is provided.

In another aspect, a storage composition is provided that includes a purified polymerase, a fluorescent DNA binding dye, and a zwitterionic detergent or non-detergent surfactant, wherein said fluorescent DNA binding dye produces a detectable signal when bound to DNA.

In still another aspect, a reaction mixture is provided that includes nucleoside-5'-triphosphates, primers, a buffer in which primer extension can occur, a polymerase, an oligonucleotide probe and a zwitterionic detergent. In this aspect, the oligonucleotide probe is operatively coupled to a detectable label.

Kits containing the compositions of the invention are also provided. In one aspect, the kit includes a polymerase and a zwitterionic detergent, but does not include a detectable label.

In another aspect, the kit includes a polymerase and two or more zwitterionic detergents, but does not include a detectable label.

In yet another aspect, the kit includes a storage buffer having a polymerase and a zwitterionic detergent. The storage buffer does not contain a detectable label.

In still another aspect, a kit which includes a storage buffer having a polymerase and a combination of two or more zwitterionic detergents is provided. The storage buffer does not contain a detectable label.

In some embodiments of the kits, the zwitterionic detergent is included in a 10X stock reaction buffer for performing PCR.

Methods of utilizing the compositions of the invention are also provided. In one aspect, a method for increasing the efficiency of a polymerase is provided. The method involves forming a reaction mixture by mixing a target nucleic acid with a polymerase, a primer, an oligonucleotide probe, a detectable label, dNTPs and a zwitterionic detergent. The detectable label is operatively coupled to the oligonucleotide probe. In some embodiments, the reaction mixture is subjected to thermal cycling.

In another aspect, a method for increasing the efficiency of a polymerase is provided. A reaction mixture is formed by mixing a target nucleic acid with a polymerase, a detectable label, a primer, an oligonucleotide probe, dNTPs and a combination of two or more zwitterionic detergents. In some embodiments, the reaction mixture is subjected to thermal cycling.

In another aspect, a method of preparing a storage composition is provided. The storage composition is formed by mixing a polymerase and a zwitterionic detergent in a suitable buffer. The storage buffer does not contain a detectable label.

In yet another aspect, a method of preparing a storage composition is provided. The storage composition is formed by mixing a polymerase and a combination of two or more zwitterionic detergents in a suitable buffer. The storage composition does not contain a detectable label.

In another aspect, a method for increasing the efficiency of a polymerase is provided. The method is performed by forming a reaction mixture which includes a target nucleic acid, a polymerase, a primer, dNTPs and a zwitterionic detergent. The reaction mixture does not contain a detectable label. In some embodiments, the reaction mixture is subjected to thermal cycling.

In yet another aspect, a method of increasing the efficiency of a polymerase is provided. The method is performed by forming a reaction mixture which includes a target nucleic acid, a polymerase, a primer, dNTPs and a combination of two or more zwitterionic detergents. The reaction mixture does not contain a detectable label. In one embodiment, the reaction mixture is subjected to thermal cycling.

In yet another aspect, a method for detecting a target nucleic acid is provided. The method includes forming a reaction mixture which includes a polymerase, primer, zwitterionic detergent, dNTPs and a detectable label; subjecting the reaction mixture to nucleic acid amplification reaction conditions, which amplify the target; and detecting a signal generated from the detectable label. The signal generated from the detectable label being indicative of the presence and/or amount of the target in the sample.

In yet another aspect, a method for detecting a target nucleic acid is provided. The method includes forming a reaction mixture which includes a polymerase, primer, zwitterionic detergent, dNTPs and an oligonucleotide probe operatively coupled to an interactive pair of labels; subjecting the reaction mixture to nucleic acid amplification reaction conditions, which amplify the target; and detecting a signal generated from a member of the interactive pair of labels. The signal generated is indicative of the presence and/or amount of the target in the sample.

Any of the above aspects may be used with a non-detergent surfactant in place of the zwitterionic detergent. Suitable non-detergent surfactants are described herein and known in the art, including, but not necessarily limited to, the Air Products series of Surfynol surfactants (Surfynol 104, Surfynol 420, Surfynol 440, Surfynol 465, Surfynol 485, Surfynol 504, Surfynol PSA series, Surfynol SE series, Dynol 604, Surfynol DF series, Surfynol CT series, and Surfynol EP series, for example Surfynol 104 series (104, 104A, 104BC, 104DPM, 104E, 104H, 104NP, 104PA, 104PG50, 104S), and Surfynol 2502).

### BRIEF DESCRIPTION OF THE DRAWINGS

Figure 1 illustrates that individual zwitterionic detergents enhance the activity of Pfu fusion DNA polymerases in PCR reactions.
Figure 2 illustrates that combinations of zwitterionic detergents enhance activity of Pfu fusion DNA polymerases.
Figure 3 further illustrates that combinations of zwitterionic detergents enhance activity of Pfu fusion DNA polymerases.
Figure 4 illustrates that zwitterionic detergents enhance the storage stability of Pfu DNA polymerases.
Figure 5 illustrates that individual zwitterionic detergents enhance the activity of Pfu DNA polymerases.
Figure 6 illustrates that zwitterionic detergents stabilize Pfu fusion DNA polymerases in accelerated stability studies.
Figure 7 illustrates that zwitterionic detergents enhance QPCR amplification with Pfu fusion DNA polymerases.
Figure 8 further illustrates that zwitterionic detergents enhance QPCR amplification with Pfu fusion DNA polymerases.
Figure 9 illustrates that Surfynol 465 enhances the activity of Pfu DNA polymerase.

### DETAILED DESCRIPTION

The invention provides compositions, kits and methods that include a polymerase and a zwitterionic detergent or non-detergent surfactant wherein said zwitterionic detergent is selected from the group consisting of: a combination of n-Decyl-N,N-dimethyl-3-ammonio-1-propanesulfate (Anzergent/Zwittergent 3-10) or n-Dodecyl-N,N-dimethyl-3-ammonio-1-propanesulfonate (Anzergent/Zwittergent 3-12) with CHAPS or CHAPSO, and
wherein said non-detergent surfactant is Surfynol 465. Such compositions and methods are useful in the storage and use of DNA polymerases in thermal cycling reactions, e.g., PCR. The present invention is based at least in part on the surprising finding that zwitterionic detergents and non-detergent surfactants increase stability and enhance activity of thermostable DNA polymerases. For example, product yields are dramatically higher when PCR amplification reactions are conducted in buffers containing one or more zwitterionic detergents (e.g., CHAPS, CHAPSO, Anzergent 3-10, and Anzergent 3-12) or non-detergent surfactants (e.g., Surfynol 465). Similarly, zwitterionic detergents and non-detergent surfactants produce higher amplification efficiencies, higher total fluorescence, and earlier Ct values in QPCR reactions employing thermostable DNA polymerase and SYBR Green to monitor duplex DNA formation.

### Zwitterionic Detergents and Non-detergent Surfactants

In one aspect, storage and reaction compositions having a polymerases and a zwitterionic detergent or non-detergent surfactant are provided. Generally, a reaction mixture will include all the necessary components to perform a nucleic acid synthesis reaction. A storage mixture may or may not include all the components necessary to perform a nucleic acid synthesis reaction.

The polymerases may be stored in a storage buffer comprising a zwitterionic detergent or non-detergent surfactant. The polymerases, described herein below, may be obtained commercially or produced by methods well known to one of ordinary skill in the art. The storage buffer and reaction buffers may include from about .001 to 5% volume/volume of each zwitterionic detergent or non-detergent surfactant employed.

As used herein, "zwitterionic detergent" refers to detergents exhibiting zwitterionic character (e.g., does not posses a net charge, lacks conductivity and electrophoretic mobility, does not bind ion-exchange resins, breaks protein-protein interactions), including, but not limited to, CHAPS and sulfobetaines sold under the brand names Zwittergent® (Calbiochem, San Diego, CA) and Anzergent® Anatrace, Inc.; Maumee, OH). Particularly suitable detergents are known in the art and described below.

Generally the zwitterionic detergent will have the general formula:

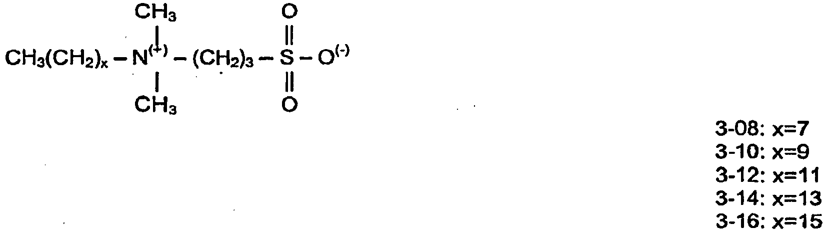

Zwitterionic detergents include those sold under the brand names Zwittergent® and Anzergent®, having the chemical names of: n-Tetradecyl-N, N- dimethyl-3-ammonio-1-propanesulfonate, n-Octyl-N,N-dimethyl-3-ammonio-1-propanesulfonate, n-Decyl-N,N-dimethyl-3-ammonio-1-propanesulfonate, and n- Dodecyl-N,N-dimethyl-3-ammonio-1-propanesulfonate. Detergents can be purchased under the brand names, for example, of: Anzergent 3-14, Analytical Grade; Anzergent 3-8, Analytical Grade; Anzergent 3-10, Analytical Grade; Anzergent 3-12, Analytical Grade, respectively or zwittergent 3-8, zwittergent 3-10, zwittergent 3-12 and zwittergent 3-14, CHAPS, CHAPSO, Apo10 and Apo12.

Preferred zwitterionic detergents include CHAPS, CHAPSO, Anzergent 3-10 and Anzergent 3-12.

As used herein, "non-detergent surfactant" refers to a composition that lowers surface tension and helps wet out surfaces, but does not have cleaning power (detergency). A "detergent" possesses cleaning power by sequestering dirt and oil in the interior of micelles formed by orienting detergent molecules with relatively small hydrophilic head groups toward the hydrophilic solvent (usually water) and hydrophobic tails (many carbon-carbon bonds, either straight chain alkyl or cyclic and/or polycyclic) toward the hydrophobic micelle interior. A non-detergent surfactant, in contrast, is a molecule with a relatively small hydrophobic head and two long hydrophilic ethylene oxide tails. The non-detergent surfactants lower surface tension but do not allow for micelle formation and detergency.

Non-detergent surfactants include, but not necessarily limited to, those sold under the brand names Surfynol 104, Surfynol 420, Surfynol 440, Surfynol 465, Surfynol 485, Surfynol 504, Surfynol PSA series, Surfynol SE series, Dynol 604, Surfynol DF series, Surfynol CT series, and Surfynol EP series, for example Surfynol 104 series (104, 104A, 104BC, 104DPM, 104E, 104H, 104NP, 104PA, 104PG50, 104S), and Surfynol 2502. Non-detergent surfactants are readily available from commercial suppliers (e.g., on the World Wide Web at airproducts.com).

Non-detergent surfactants include, but not necessarily limited to, the DOWFAX series ofNonionic surfactants that are produced by polymerizing ethylene oxide (EO), propylene oxide (PO), and/or butylene oxide (BO) in the same molecule. These include those sold under the brand name, DOWFAX 63N10, DOWFAX 63N13, DOWFAX 63N30, DOWFAX 63N40, DOWFAX 81N13, DOWFAX 81N15, DOWFAX 92N20, DOWFAX 100N15, DOWFAX EM-51, DOWFAX 20A42, DOWFAX 20A64, DOWFAX 20A612, DOWFAX 20B102, DOWFAX DF-101, DOWFAX DF-111, DOWFAX DF-112, DOWFAX DF-113, DOWFAX DF-114, DOWFAX DF-117, DOWFAX WP-310, DOWFAX 50C15, DOWFAX DF-121, DOWFAX DF-122, DOWFAX DF-133, DOWFAX DF-141, DOWFAX DF-142, DOWFAX DF-161) (DOW Chemical Company, Midland, Michigan).

Non-detergent surfactants include, but not necessarily limited to, the PLURONIC^{®} block copolymer series of surfactants having the general structure (C₂H₄O)ₐ(C3H60)_{b}(C₂H₄O)ₐH]. These include those sold under the brand name, PLURONIC^{®} block copolymer series of surfactants (, L35, P65, P75, P85, P103, P104, P105, F108) (BASF Corporation; Mount Olive, NJ).

Other non-detergent surfactants include: Dimethylethylammonium-1-propanesulfonate, 3-(1-Pyridino)-1-propanesulfonate, Dimethyl-2-hydroxyethyl-1 - propanesulfonate, 3-(1 -Methylpiperidinium)-1-propanesulfonate, N-Methyl-N-(3-sulfopropyl)morpholinium and Dimethylbenzylammonium-1-propanesulfonate.

The zwitterionic detergent or non-detergent surfactant is used in an amount effective to induce the desired result (e.g., stabilize and/or enhance activity of a thermostable DNA polymerase). The optimal concentration of zwitterionic detergent or non-detergent surfactant for use in the compositions and methods will often vary between polymerases. One of skill in the art may perform routine testing to determine the optimal concentration of zwitterionic detergent or non-detergent surfactant for use with the particular polymerase. For example, a series of PCR reactions can be performed in which only the concentration of the detergent is varied (e.g., .05% to 1% Anzergent 3-10). The polymerase activity can then be determined by detecting and/or quantifying the amplified product by methods known in the art and described herein, (e.g., quantification by real-time PCR or gel electrophoresis of amplified product; See Examples 1-5). The most effective concentration of the zwitterionic detergent or non-detergent surfactant for use with the polymerase is the concentration which results in the most amplified product.

Similarly, test zwitterionic or non-detergent surfactants can be assayed for their effectiveness in amplification reactions by performing the assay described above and comparing the amount of amplified product produced in the composition comprising the test Zwitterionic detergent or non-detergent surfactant to a negative control that does not include any surfactant.

The effectiveness of zwitterionic and non-detergent surfactants in stabilizing polymerases in a storage compositions can be assayed by similar methods. For example, the storage stability studies may be performed by storing the polymerase with the zwitterionic or non-detergent surfactant for a period of time (e.g., 1 week) at -20°C. The polymerase is then assayed for its ability to amplify a target nucleic acid as described above. Alternatively, an accelerated stability test may be preformed in which the polymerase and zwitterionic detergent or non-detergent surfactant to be tested is subjected to 95°C for 6 hours. The polymerase is then assayed for its ability to amplify a target nucleic acid and a comparison is made of the amount of amplified product in the reaction utilizing the zwitterionic detergent or non-detergent surfactant to a reaction mixture that is surfactant free. If the amplified product is greater with tested surfactant then the zwitterionic detergent/non-detergent surfactant than the surfactant free reaction is effective at stabilizing the polymerase in a storage composition (See Example 3)

In one embodiment, the zwitterionic detergent is CHAPS. In a further embodiment, CHAPS is present at a concentration of about .05-1.0% volume/volume of the total composition. In a further embodiment, CHAPS is present at a concentration of about .2-.8% volume/volume of the total composition. In yet a further embodiment, CHAPS is present at a concentration of about .2-.4% volume/volume of the total composition.

In another embodiment, CHAPSO is present at a concentration of about .05-1.0% volume/volume of the total composition. In yet another embodiment, CHAPSO is present at a concentration of about .1-.4% volume/volume of the total composition. In a further embodiment, CHAPSO is present at a concentration of about .15-.35% volume/volume of the total composition.

In yet another embodiment, Anzergent 3-10 is present at a concentration of about .1-1.0% volume/volume of the total composition. In a further embodiment, Anzergent 3-10 is present at a concentration of about .4-.8% volume/volume of the total composition.

In still another embodiment, Anzergent 3-12 is present at a concentration of about .05-1.0% volume/volume of the total composition. In still another embodiment, Anzergent 3-12 is present at a concentration of about 1-.4% volume/volume of the total composition. In a further embodiment, Anzergent 3-12 is present at a concentration of about 1-.2% volume/volume of the total composition.

It is also envisioned that compatible zwitterionic detergents can be mixed together to provide the requisite detergent. Generally, any two different zwitterionic or non-detergent surfactants may be present in a ratio of 1:1, 1:2, 1:5, 1:10, 1:100, 100:1, 10:1, 5:1 or 2:1. For example, the composition may include a combination of CHAPS and Anzergent 3-12; CHAPS. and Anzergent 3-10; CHAPSO and Anzergent 3-12; or CHAPSO and Anzergent 3-10.

In one embodiment, CHAPS is present at a concentration of about .1% and Anzergetn 3-12 is present at a concentration of .1% to .5%. In yet another embodiment, CHAPSO is present at a concentration of .1% and Anzergent 3-10 is present at a concentration of .05% to .5%. In a further embodiment CHAPSO is present at a concentration of .1% and Anzergent 3-10 is present at a concentration of .05% to .4%. In yet another embodiment, CHAPSO is present at a concentration of .05% to .1% and Anzergent 3-12 is present at a concentration of .05% to .5%. %. In a further embodiment, CHAPSO is present at a concentration of .05% to .1% and Anzergent 3-12 is present at a concentration of .05% to .4%. Additional zwitterionic detergent concentrations are illustrated in the Examples.

In one aspect, a storage composition having a polymerase and a zwitterionic detergent or non-detergent surfactant is provided. In another aspect, a storage composition which includes a polymerase and a combination of two or more zwitterionic detergents or non-detergent surfactants is provided. In these aspects, the storage composition does not contain a detectable label.

In one embodiment, the storage buffer comprises Tris-HCL or TrisSO₄, and a pH of about 8-10. In a further embodiment, the storage buffer includes 50% (v/v) glycerol, 50 mM TrisHCl (pH 8.2), 0.1 mM ethylenediaminetetraacetic acid (EDTA), and 1 mM dithiothreitol.

In another embodiment, the storage buffer includes 20mM TrisHCL (pH8.8), 10mM KCL, 10mM (NH₄)₂SO₄, 2mM MgSO₄ and 100ug/ml BSA. In yet another embodiment, the storage buffer includes 40mM TrisSO₄ (pH10), 15mM K₂SO₄, 8mM (NH₄)₂SO₄, and 2mM MgSO₄. In still another embodiment, the storage buffer includes 30mM TrisSO₄ (pH 10), 40mM K₂SO₄, 1.5mM (NHa)₂SO₄, and 2mM MgSO₄. Other suitable storage buffers are contemplated and are known in the art.

In another aspect, a storage composition that includes a purified polymerase, a labeled nucleotide, and a zwitterionic detergent or non-detergent surfactant is provided. In one embodiment, the labeled nucleotide has a single detectable label. For example, the single detectable label may be a fluorophore. In another embodiment, the labeled nucleotide has an interactive pair of labels. Suitable interactive pair of labels include a quencher and a fluorophore.

In another aspect, a storage composition is provided that includes a purified polymerase, a fluorescent DNA binding dye, and a Zwitterionic detergent or non-detergent surfactant, wherein said fluorescent DNA binding dye produces a detectable signal when bound to DNA. Suitable DNA binding dyes are known in the art and described herein. For example, DNA binding dyes include, but are not limited to, SYBR Green or EvaGreen.

In another aspect, a reaction mixture which includes a polymerase and zwitterionic detergent or non-detergent surfactant is provided. The reaction buffer is useful for the amplification of a target nucleic acid. The reaction buffer comprises from about ...001 to 5% volume/volume of each zwitterionic detergent or non-detergent surfactant employed.

In one aspect, a reaction mixture that includes a polymerase, an oligonucleotide probe and a zwitterionic detergent or non-detergent surfactant is provided.

In another aspect, a reaction mixture is provided that includes a polymerase, a detectable label, and a combination of two or more zwitterionic detergents or non-detergent surfactants or a combination thereof.

In one embodiment, the detectable label is operatively coupled to the oligonucleotide probe.

In another embodiment, the detectable label comprises an interactive pair of labels.

In still another aspect, a reaction mixture is provided that includes nucleoside-5'-triphosphates, primers, a buffer in which primer extension can occur, a polymerase, an oligonucleotide probe and a zwitterionic detergent.

In one embodiment, the oligonucleotide probe is operatively coupled to a detectable label. In another embodiment, the detectable label comprises an interactive pair of labels.

In another aspect, a reaction mixture having a purified polymerase, a labeled nucleotide, and a zwitterionic detergent or non-detergent surfactant is provided. In one embodiment the labeled nucleotide has a single detectable label. For example, the single detectable label may be a fluorophore. In another embodiment, the labeled nucleotide has an interactive pair of labels. A suitable interactive pair of labels includes a quencher and a fluorophore.

In another aspect, the reaction mixture includes a purified polymerase, a fluorescent DNA binding dye, and a zwitterionic detergent or non-detergent surfactant, wherein said fluorescent DNA binding dye produces a detectable signal when bound to DNA. Suitable DNA binding dyes are known in the art and described herein. For example, DNA binding dyes include, but are not limited to, SYBR Green or EvaGreen.

In one embodiment, the reaction mixture comprises a buffer having Tris-HCl or Tris-SO₄, KCL or K₂SO₄ a pH of about 8.0 to pH10, (NH₄)₂SO₄ and MgSO₄. In a further embodiment, the reaction mixture includes Tris-HCl (pH 8.8), KCl, (NH₄)₂SO₄ and MgSO₄.

In another embodiment, the reaction buffer includes 20mM TrisHCL (pH8.8), 10mM KCL, 10mM (NH₄)₂SO₄, 2mM MgSO₄ and 100ug/ml BSA. In yet another embodiment, the reaction buffer includes 40mM TrisSO₄ ((pH10), 15mM K₂SO₄, 8mM (NH₄)₂SO₄, and 2mM MgSO₄. In still another embodiment, the reaction buffer includes 30mM TrisSO₄ (pH 10), 40mM K₂SO₄, 1.5mM (NH4)₂SO₄, and 2mM MgSO₄.

Additional storage and reaction buffers useful in practicing the invention are known in the art (e.g., those described in U.S. Publication No. 2005/0048530, filed March 14,2004; U.S. Application No. 11/152,773, filed 06/15/2006; and U.S. Application No. 60/757,120, filed January 6, 2006) and described in the Examples.

For example, a composition may include a thermostable DNA polymerase, the buffer described in U.S. Application No. 11/152,773, which comprises tris(2-carboxyethyl)phosphine (TCEP) or similar phosphine compounds, and a non-ionic surfactant. In this embodiment, the non-ionic surfactant is a non-detergent non-ionic surfactant such as the Surfynol series of surfactants.

In another embodiment, the composition includes a thermostable polymerase, the buffer described in U.S. Application No. 60/757,120, a zwitterionic or non-detergent surfactant (e.g., Surfynol series) and a buffer comprising potassium sulfate and ammonium sulfate which has a potassium sulfate:ammonium sulfate molar ratio of 5:1 to 50:1. In some embodiments, the potassium sulfate concentration ranges from 20 mM to 50 mM, and the ammonium sulfate concentration ranges from 1 to 5 mM.

The buffer for use in the compositions and methods described will be suitable for the particular polymerase. Suitable buffers are known in the art and described in the literature provided by the commercial source of the polymerase.

Any of the above listed aspects may be performed with a non-detergent surfactant or zwitterionic detergent. Suitable non-detergent surfactants include the Air Products series of Surfynol surfactants, including, but not necessarily limited to, Surfynol 104, Surfynol 420, Surfynol 440, Surfynol 465, Surfynol 485, Surfynol 504, Surfynol PSA series, Surfynol SE series, Dynol 604, Surfynol DF series, Surfynol CT series, and Surfynol EP series, for example Surfynol 104 series (104, 104A, 104BC, 104DPM, 104E, 104H, 104NP, 104PA, 104PG50, 104S), and Surfynol 2502. Non-detergent surfactants are readily available from commercial suppliers (e.g., on the World Wide Web at airproducts.com).

### Polymerases

The zwitterionic detergent/non-detergent surfactant is used in combination with the nucleic acid polymerase. As used herein, "nucleic acid polymerase" or "polymerase" refers to an enzyme that catalyzes the polymerization of nucleotides. Generally, the enzyme will initiate synthesis at the 3'-end of the primer annealed to a nucleic acid template sequence, and will proceed in the 5'-direction along the template. "DNA polymerase" catalyzes the polymerization of deoxynucleotides. Known DNA polymerases include, for example, *Pyrococcus furiosus* (Pfu) DNA polymerase, *E. coli* DNA polymerase I, T7 DNA polymerase, *Thermus thermophilus* (Tth) DNA polymerase, *Bacillus stearothermophilus* DNA polymerase, *Thermococcus litoralis* (Tli) DNA polymerase (also referred to as Vent DNA polymerase), *Thermotoga maritima* (UlTma) DNA polymerase, *Thermus aquaticus* (Taq) DNA polymerase, and *Pyrococcus GB-D* (PGB-D) DNA polymerase.

DNA polymerases and their properties are described in detail in, among other places, DNA Replication 2nd edition, Kornberg and Baker, W. H. Freeman, New York, N.Y. (1991). Known conventional DNA polymerases include, for example, *Pyrococcus furiosus* (Pfu) DNA polymerase (Lundberg et al., 1991, Gene 108:1, provided by Stratagene, La Jolla, California, USA), *Pyrococcus woesei* (Pwo) DNA polymerase (Hinnisdaels et al., 1996, Biotechniques 20:186-8), *Thermus thermophilus* (Tth) DNA polymerase (Myers and Gelfand 1991, Biochemistry 30:7661), *Bacillus stearothermophilus* DNA polymerase (Stenesh and McGowan, 1977, Biochim. Biophys. Acta 475:32), *Thermococcus litoralis* (Tli) DNA polymerase (also referred to as Vent DNA polymerase, Cariello et al., 1991, Polynucleotide Res. 19: 4193, available from, *e.g.*, New England Biolabs, Beverly, Massachusetts, USA), 9°Nm DNA polymerase, *Thermotoga maritima* (Tma) DNA polymerase (Diaz and Sabino, 1998. Braz. J. Med. Res. 31:1239), *Thermus aquaticus* (Taq) DNA polymerase (Chien et al., 1976, J. Bacteoriol. 127:1550), *Pyrococcus kodakaraensis* KOD DNA polymerase (Takagi et al., 1997, Appl. Environ. Microbiol. 63:4504), JDF-3 DNA polymerase (from *Thermococcus sp.* JDF-3, Published International patent application WO 01/32887), *Pyrococcus GB-D* (CPGB-D) DNA polymerase (also referred as DeepVent DNA polymerase, Juncosa-Ginesta et al., 1994, Biotechniques 16:820, available from, e.g., New England Biolabs, Beverly, Massachusetts, USA), UlTma DNA polymerase (from thermophile *Thermotoga maritima*; Diaz and Sabino, 1998, Braz. J. Med. Res. 31:1239; available from, e.g., PE Applied Biosystems, Foster City, California, USA), Tgo DNA polymerase (from *Thermococcus gorgonarius*, available from, *e.g.*, Roche Molecular Biochemicals, Indianapolis, Indiana, USA), *E. coli* DNA polymerase I (Lecomte and Doubleday, 1983, Polynucleotide Res. 11:7505), T7 DNA polymerase (Nordstrom et al., 1981, J. Biol. Chem. 256:3112), and archaeal DP1/DP2 DNA polymerase II (Cann et al., 1998, Proc. Natl. Acad. Sci. USA 95:14250-5).

In one embodiment, the polymerase is a purified polymerase. As used herein, "purified" refers to a polymerase, which has been separated from components which naturally accompany it.

The term, "nucleic acid polymerase" also encompasses reverse transcriptases including, but not limited to, reverse transcriptases from HIV, HTLV-1, HTLV-II, FeLV, FIV, SIV, AMV, MMTV, MoMuLV and other retroviruses (for reviews, see for example, Levin, 1997, Cell 88:5-8; Verma, 1977, Biochim. Biophys. Acta 473:1-38; Wu et al., 1975, CRC Crit. Rev. Biochem. 3:289-347).

When using the subject compositions in reaction mixtures that are exposed to elevated temperatures (*e.g.*, during the PCR technique) use of thermostable DNA polymerases is preferred. As used herein, "thermostable" refers to a property of a nucleic acid polymerase, such that the enzyme is active at elevated temperatures and is resistant to nucleic acid duplex-denaturing temperatures in the range of about 93°C to about 100 °C. "Active" means the enzyme retains the ability to effect primer extension reactions when subjected to elevated or denaturing temperatures for the time necessary to effect denaturation of double-stranded nucleic acids. Elevated temperatures as used herein refer to the range of about 70°C to about 100 °C, whereas non-elevated temperatures as used herein refer to the range of about 35°C to about 50 °C.

Thermostable DNA polymerases that may be used in the invention include Taq, Tne, Tma, *Pfu*, Tfl, Tth, Stoffel fragment, VENT™ and DEEPVENT™ DNA polymerases, KOD, Tgo, JDF3, and mutants, variants and derivatives thereof (U.S. Pat. No. 5,436,149; U.S. Patent 4,889,818; U.S. Pat. No. 4,965,185; U.S. Pat. No. 5,079,352; U.S. Patent 5,614,365; U.S. Pat. No. 5,374,553; U.S. Pat. No. 5,270,179; U.S. Pat. No. 5,047,342; U.S. Pat. No. 5,512,462; WO 92/06188; WO 92/06200; WO 96/10640; Barnes, W. M., Gene 112:29-35 (1992); Lawyer, F. C., et al., PCR Meth. Appl. 2:275-287 (1993); Flaman, J. -M, et al., Nuc. Acids Res. 22(15):3259- 3260 (1994)).

In one embodiment, the thermostable DNA polymerase is a Pfu DNA polymerase or a Taq DNA polymerase. In a further embodiment, the thermostable DNA polymerase is Pfu DNA polymerase with a mutation at position V93 and wherein the polymerase is exonuclease deficient (e.g., Pfu V93, exo-). Methods of making and using Pfu V93, exo- DNA polymerase are described in U.S. Patent Application No.: 10/298,680, filed November 18, 2002. In another embodiment, the polymerase is a fusion protein having polymerase activity (e.g., Pfu DNA polymerase-Sso7, as described in U.S. Patent Application No.: 60/699,937, filed July 15, 2005 and U.S. Publication No. 2005/0048530, filed March 14, 2004.

### Applications

The zwitterionic detergent/non-detergent surfactant and polymerase compositions described herein may be used in any application for which polymerases are known to be used (e.g., nucleic acid amplification, PCR, QPCR, sequencing mutagenesis).

As used herein, the term "nucleic acid amplification" refers to the production of additional copies of a nucleic acid sequence and is generally carried out using polymerase chain reaction (PCR) or ligase chain reaction (LCR) technologies well known in the art (Dieffenbach, C. W. and G. S. Dveksler (1995) PCR Primer, a Laboratory Manual, Cold Spring Harbor Press, Plainview, N.Y.).

As used herein, the term "polymerase chain reaction" or "PCR" refers to an in vitro method for amplifying a specific polynucleotide template sequence. The technique of PCR is described in numerous publications, including, PCR: A Practical Approach, M. J. McPherson, et al., IRL Press (1991), PCR Protocols: A Guide to Methods and Applications, by Innis, et al., Academic Press (1990), and PCR Technology: Principals and Applications for DNA Amplification, H. A. Erlich, Stockton Press (1989). PCR is also described in many U.S. Patents, including U.S. Patent Nos. 4,683,195; 4,683,202; 4,800,159; 4,965,188; 4,889,818; 5,075,216; 5,079,352; 5,104,792; 5,023,171; 5,091,310; and 5,066,584.

For ease of understanding the advantages provided by the present invention, a summary of PCR is provided. The PCR reaction involves a repetitive series of temperature cycles and is typically performed in a volume of 50-100 ul. The reaction mix comprises dNTPs (each of the four deoxynucleotides dATP, dCTP, dGTP, and dTTP), primers, buffers, DNA polymerase, and polynucleotide template. PCR requires two primers that hybridize with the double-stranded target polynucleotide sequence to be amplified. In PCR, this double-stranded target sequence is denatured and one primer is annealed to each strand of the denatured target. The primers anneal to the target polynucleotide at sites removed from one another and in orientations such that the extension product of one primer, when separated from its complement, can hybridize to the other primer. Once a given primer hybridizes to the target sequence, the primer is extended by the action of a DNA polymerase. The extension product is then denatured from the target sequence, and the process is repeated.

In successive cycles of this process, the extension products produced in earlier cycles serve as templates for DNA synthesis. Beginning in the second cycle, the product of amplification begins to accumulate at a logarithmic rate. The amplification product is a discrete double-stranded DNA molecule comprising: a first strand which contains the sequence of the first primer, eventually followed by the sequence complementary to the second primer, and a second strand which is complementary to the first strand.

In one aspect, a method for increasing the efficiency of a polymerase is provided. The method involves forming a reaction mixture by mixing a target nucleic acid with a polymerase, a primer, an oligonucleotide probe, a detectable label, dNTPs and a zwitterionic detergent or non-detergent surfactant. In one embodiment, the detectable label is operatively coupled to the oligonucleotide probe. In a further embodiment, the reaction mixture is subjected to thermal cycling. Thermal cycling is subjecting a reaction mixture to two or more different incubation temperatures for a period of time. For example, the denaturing (e.g., 95°C for 1 minute) and annealing/extension (65°C for 30s) phases of an nucleic acid amplification reaction.

In another aspects, a method is provided for increasing the efficiency of a polymerase by forming a reaction mixture by mixing a target nucleic acid with a polymerase, a detectable label, a primer, an oligonucleotide probe, dNTPs and a combination of two or more zwitterionic detergents or non-detergent surfactants. In one embodiment, the reaction mixture is subjected to thermal cycling.

In another aspect, a method for increasing the efficiency of a polymerase is provided. The method is performed by forming a reaction mixture which includes a target nucleic acid, a polymerase, a primer, dNTPs and a zwitterionic detergent or non-detergent surfactant. In this embodiment the reaction mixture does not contain a detectable label. In a further embodiment, the reaction mixture is subjected to thermal cycling.

In yet another aspect, a method of increasing the efficiency of a polymerase by forming a reaction mixture which includes a target nucleic acid, a polymerase, a primer, dNTPs and a combination of two or more zwitterionic detergents or non-detergent surfactants or combination thereof is provided. The reaction mixture does not contain a detectable label. In one embodiment, the reaction mixture is subjected to thermal cycling.

In yet another aspect, a method of increasing the efficiency of a polymerase by forming a reaction mixture which includes a target nucleic acid, a purified polymerase, a primer, a detectable label, nucleoside-5'-triphosphates and a zwitterionic detergent or non-detergent surfactant is provided. In one embodiment, the detectable label is a labeled nucleotide. In a further embodiment, the labeled nucleotide has a single detectable label. For example, the single detectable label may be a fluorophore. In another embodiment, the labeled nucleotide has an interactive pair of labels. A suitable interactive pair of labels includes a quencher and a fluorophore.

In another embodiment, the detectable label is a fluorescent DNA binding dye, wherein the fluorescent DNA binding dye produces a detectable signal when bound to DNA. Suitable DNA binding dyes are known in the art and described herein. For example, DNA binding dyes include, but are not limited to, SYBR Green or EvaGreen..

In another aspect, a method for detecting a target nucleic acid is provided. The method includes forming a reaction mixture which includes a polymerase, primer, zwitterionic detergent or non-detergent surfactant, dNTPs and a detectable label; subjecting the reaction mixture to nucleic acid amplification reaction conditions, which amplify the target; and detecting a signal generated from the detectable label. The signal generated from the detectable label being indicative of the presence and/or amount of the target in the sample. In one embodiment, the reaction mixture further includes an oligonucleotide probe. In yet a further embodiment, the oligonucleotide probe and detectable label are operatively coupled. In another embodiment, the detectable label is an intercalating detectable label (e.g., SYBR green).

In yet another aspect, a method for detecting a target nucleic acid is provided. The method includes forming a reaction mixture which includes a polymerase, primer, zwitterionic detergent or non-detergent surfactant, dNTPs and an oligonucleotide probe operatively coupled to an interactive pair of labels; subjecting the reaction mixture to nucleic acid amplification reaction conditions, which amplify the target; and detecting a signal generated from a member of the interactive pair of labels. The signal generated being indicative of the presence and/or amount of the target in the sample.

As used herein, "nucleic acid amplification reaction conditions" refer to a buffer, a set of temperature incubation steps and times that are possible and preferably optimal for conducting amplification of a nucleic acid. Amplification means an increase in the number of a particular nucleic acid sequence and may be accomplished, without limitation, by the *in vitro* methods of polymerase chain reaction or ligase chain reaction. Such reaction conditions are known in the art and are described herein. Nucleic acid reaction conditions encompass PCR reaction conditions.

In one embodiment, the step of subjecting the reaction mixture to nucleic acid amplification reaction conditions includes the step of heating the reaction mixture with a thermal cycler sample block so as to denature the target nucleic acid.

In one embodiment, the oligonucleotide probe is cleaved by a 5' nuclease during the amplification reaction. In yet a further embodiment, the probe is cleaved separating the members of the interactive pair of labels and generating a detectable signal. Such methods are known in the art and described in U.S. Patent Nos.: 6,528,254; 6,548,250 and; 5,210,015.

In another aspect, the zwitterionic detergent or non-detergent surfactant is used in a mutagenesis reaction. For example, a zwitterionic detergent or non-detergent surfactant may be used in place of Triton-x 100 in the QUICKCHANGE site directed mutagenesis kit (Stratagene catalog #200518).

### Labels

It is contemplated that the invention will often include detectable labels. The detectable labels may be operatively coupled to the probe (e.g., FAM and BHQ2), may be provided free in solution (e.g., fluorescent DNA binding dyes, SYBR green), or operatively coupled to a nucleotide precursor.

The use of labeled probes in the amplification and quantification of a target polynucleotide (e.g., PCR) is described in many references, such as Innis et al., editors, PCR Protocols (Academic Press, New York, 1989); Sambrook et al., Molecular Cloning, Second Edition (Cold Spring Harbor Laboratory, New York, 1989).

As used herein, the term "probe" or "oligonucleotide probe" refers to a single-stranded oligonucleotide having a sequence partly or completely complementary to a nucleic acid sequence sought to be detected, so as to stably hybridize thereto under stringent hybridization conditions. Probes may, but need not, have regions which are not complementary to a target sequence, as long as such sequences do not substantially alter the probe's desired specificity under stringent hybridization conditions.

In some embodiments, the probe is operatively coupled to a "label". As used herein, the term "label" refers to any atom or molecule which can be used to provide a detectable (preferably quantifiable) signal, and which can be operatively linked to a nucleic acid. Labels may provide signals detectable by fluorescence, radioactivity, colorimetry, gravimetry, X-ray diffraction or absorption, magnetism, enzymatic activity, mass spectrometry, binding affinity, hybridization radiofrequency and the like.

In further embodiments, the probe is operatively coupled to an interactive pair of labels. As used herein, the phrase "interactive pair of labels" as well as the phrase "pair of interactive labels" as well as the phrase "first member and second member" refer to a pair of molecules which interact physically, optically, or otherwise in such a manner as to permit detection of their proximity by means of a detectable signal. Examples of a "pair of interactive labels" include, but are not limited to, labels suitable for use in fluorescence resonance energy transfer (FRET) (Stryer, L. Ann. Rev. Biochem. 47, 819-846, 1978), scintillation proximity assays (SPA) (Hart and Greenwald, Molecular Immunology 16:265-267, 1979; U.S. Pat. No. 4,658,649), luminescence resonance energy transfer (LRET) (Mathis, G. Clin. Chem. 41, 1391-1397, 1995), direct quenching (Tyagi et al., Nature Biotechnology 16, 49-53, 1998), chemiluminescence energy transfer (CRET) (Campbell, A. K., and Patel, A. Biochem. J. 216, 185-194, 1983), bioluminescence resonance energy transfer (BRET) (Xu, Y., Piston D. W., Johnson, Proc. Natl. Acad. Sc., 96, 151-156, 1999), or excimer formation (Lakowicz, J. R. Principles of Fluorescence Spectroscopy, Kluwer Academic/Plenum Press, New York, 1999).

The pair of labels can be either covalently or non-covalently attached to the oligonucleotide probes.

A pair of interactive labels useful for the invention can comprise a pair of FRET-compatible detectable labels, or a quencher-detectable label pair. In one embodiment, the pair comprises a fluorophore-quencher pair.

A wide variety of fluorophores can be used, including but not limited to: 5 - FAM (also called 5 - carboxyfluorescein; also called Spiro(isobenzofuran - 1(3H), 9' - (9H)xanthene) - 5 - carboxylic acid,3',6' - dihydroxy - 3 - oxo - 6 - carboxyfluorescein); 5 - Hexachloro - Fluorescein ([4,7,2',4',5',7' - hexachloro-(3',6' - dipivaloyl - fluoresceinyl) - 6 - carboxylic acid]); 6 - Hexachloro - Fluorescein ([4,7,2',4',5',7' - hexachloro - (3',6' - dipivaloylfluoresceinyl) - 5 - carboxylic acid]); 5,- Tetrachloro - Fluorescein ([4,7,2',7' - tetra - chloro - (3',6' - dipivaloylfluoresceinyl) - 5 - carboxylic acid]); 6 - Tetrachloro - Fluorescein ([4,7,2',7' - tetrachloro - (3',6' - dipivaloylfluoresceinyl) - 6 - carboxylic acid]); 5 - TAMRA (5 - carboxytetramethylrhodamine; Xanthylium, 9 - (2,4 - dicarboxyphenyl) - 3,6 - bis(dimethyl - amino); 6 - TAMRA (6 - carboxytetramethylrhodamine; Xanthylium, 9 - (2,5 - dicarboxyphenyl) - 3, 6 - bis(dimethylamino); EDANS (5 - ((2 - aminoethyl) amino)naphthalene - 1 - sulfonic acid); 1,5 - IAEDANS (5 - ((((2 - iodoacetyl)amino)ethyl) amino)naphthalene - 1 - sulfonic acid); DABCYL (4 - ((4 - (dimethylamino)phenyl) azo)benzoic acid) Cy5 (Indodicarbocyanine - 5) Cy3 (Indo - dicarbocyanine - 3); and BODIPY FL (2,6 - dibromo - 4,4 - difluoro - 5,7 - dimethyl - 4 - bora - 3a,4a - diaza - s - indacene - 3 - proprionic acid), Quasar-670 (Biosearch Technologies), CalOrange (Biosearch Technologies), Rox, as well as suitable derivatives thereof.

As used herein, the term "quencher" refers to a chromophoric molecule or part of a compound, which is capable of reducing the emission from a fluorescent donor when attached to or in proximity to the donor. Quenching may occur by any of several mechanisms including fluorescence resonance energy transfer, photo-induced electron transfer, paramagnetic enhancement of intersystem crossing, Dexter exchange coupling, and exciton coupling such as the formation of dark complexes. Fluorescence is "quenched" when the fluorescence emitted by the fluorophore is reduced as compared with the fluorescence in the absence of the quencher by at least 10%, for example,15%, 20%, 30%, 40%, 50%, 60%, 70%, 80%, 90%, 95%, 98%, 99%, 99.9% or more.

The quencher can be any material that can quench at least one fluorescence emission from an excited fluorophore being used in the assay. There is a great deal of practical guidance available in the literature for selecting appropriate reporter-quencher pairs for particular probes, as exemplified by the following references: Clegg (1993, Proc. Natl. Acad. Sci., 90:2994-2998); Wu et al. (1994, Anal. Biochem., 218:1-13); Pesce et al., editors, Fluorescence Spectroscopy (1971, Marcel Dekker, New York); White et al., Fluorescence Analysis: A Practical Approach (1970, Marcel Dekker, New York); and the like. The literature also includes references providing exhaustive lists of fluorescent and chromogenic molecules and their relevant optical properties for choosing reporter-quencher pairs, e.g., Berlman, Handbook of Fluorescence Spectra of Aromatic Molecules, 2nd Edition (1971, Academic Press, New York); Griffiths, Colour and Constitution of Organic Molecules (1976, Academic Press, New York); Bishop, editor, Indicators (1972, Pergamon Press, Oxford); Haugland, Handbook of Fluorescent Probes and Research Chemicals (1992 Molecular Probes, Eugene) Pringsheim, Fluorescence and Phosphorescence (1949, Interscience Publishers, New York). Further, there is extensive guidance in the literature for derivatizing reporter and quencher molecules for covalent attachment via common reactive groups that can be added to an oligonucleotide, as exemplified by the following references, see, for example, Haugland (cited above); Ullman et al., U.S. Pat. No. 3,996,345; Khanna et al., U.S. Pat. No. 4,351,760.

A number of commercially available quenchers are known in the art, and include but are not limited to DABCYL, BHQ-1, BHQ-2, and BHQ-3. The BHQ ("Black Hole Quenchers") quenchers are a new class of dark quenchers that prevent fluorescence until a hybridization event occurs. In addition, these new quenchers have no native fluorescence, virtually eliminating background problems seen with other quenchers. BHQ quenchers can be used to quench almost all reporter detectable labels and are commercially available, for example, from Biosearch Technologies, Inc (Novato, CA).

Appropriate linking methodologies for attachment of many detectable labels to oligonucleotides are described in many references, e.g., Marshall, Histochemical J., 7: 299-303 (1975); Menchen et al., U.S. Pat. No. 5,188,934; Menchen et al., European Patent Application 87310256.0; and Bergot et al., International Application PCT/US90/05565.

In some embodiments, the compositions described includes a detectable label. The detectable label may be any detectable label which will produce a signal indicative of the presence or amount of a target nucleic acid. Such detectable labels are known in the art and described above. Detectable labels useful according to the invention include fluorescent detectable labels such as SYBR green and FAM. In one embodiment, the label does not convert electromagnetic energy into thermal energy in order to heat the reaction mixture (e.g., as described in U.S. Publication No.: US 2003/0017567).

In one embodiment, the label is operatively coupled to a nucleotide. In one embodiment, the labeled nucleotide is a dual labeled nucleotides, as described in U.S. Publication No. 2004/0014096, filed April 4, 2003. The dual labeled nucleotide includes a fluorescent label and a quencher of that fluorescent label.

Other suitable dual labeled nucleotides include, for example, those taught in Rosenblum et al. (1997, Nucleic Acids Research, 25: 4500). Rosenblum et al. teaches the use of nucleotide analogs comprising a fluorescence resonance energy transfer (FRET) dye pair linked to the nucleobase. Incorporation of such analogs into a growing polynucleotide chain is detected by contacting the analog with light of a wavelength within the excitation spectrum of one of the dyes but not the other. The light emitted by the excited fluorophore then, in turn, excites the second dye, from which fluorescence emission is detected. In addition, Williams (U.S. Pat. App. Pub. 20010018184) teaches a dual-labeled nucleotide analog in which a fluorophore is attached to the gamma.-phosphate of the polyphosphate moiety, and a quencher is linked elsewhere on the nucleotide analog, preferably linked to the 5' carbon of pyrimidine bases and to the 7' carbon of deazapurine bases. Upon incorporation of the analog taught by Williams into a growing polynucleotide chain, the phosphate group linked to the fluorescent moiety is cleaved off, thus separating the fluorescent moiety and the quencher, thereby permitting the fluorescent moiety to emit a detectable signal.

### Kits

Compositions and methods having a zwitterionic detergent/non-detergent surfactant and a polymerase as described herein are provided. A kit format is also contemplated which comprises a package unit having one or more containers of the subject composition and in some embodiments including containers of various reagents used for polynucleotide synthesis, including synthesis in PCR, sequencing and mutagenesis. The kit may also contain one or more of the following items: polynucleotide precursors (e.g., nucleoside triphosphates), primers, probes, buffers, instructions, labeled nucleotides, intercalating dyes and controls. The kits may include containers of reagents mixed together in suitable proportions for performing the methods in accordance with the invention. Reagent containers preferably contain reagents in unit quantities that obviate measuring steps when performing the subj ect methods. One kit according to the invention also contains a DNA yield standard for the quantitation of the PCR product yields from a stained gel. In one embodiment, the kit includes a master mix reagent comprising the thermostable polymerase a zwitterionic or non-detergent surfactant and polynucleotide precursors.

In one embodiment, the kit includes a storage and/or reaction buffer having a polymerase and a zwitterionic detergent or non-detergent surfactant. The storage buffer does not contain a detectable label.

In another embodiment, a kit which includes a storage and/or reaction buffer having a polymerase and a combination of two or more zwitterionic detergents is provided. The storage buffer does not contain a detectable label.

In yet another embodiment, the kits may further include a separate container having dNTPs. In another embodiment, any of the above kits may further include a separate container having a detectable label.

In another aspect, a kit is provided which includes a purified polymerase, a zwitterionic detergent or non-detergent surfactant, polynucleotide precursors and a labeled nucleotide. In yet another aspect, a kit which includes a purified polymerase, a zwitterionic detergent or non-detergent surfactant, polynucleotide precursors and a DNA binding dye is provided.

In some embodiments of the kits, the zwitterionic detergent or non-detergent surfactant is provided as in a 10X concentration in a 10X stock reaction buffer that is suitable for performing a nucleic acid amplification reaction.

### EXAMPLES

### Example 1--Preparing Polymerases and Buffers Lacking Conventional Non-Ionic Detergents.

Pfu (exo+ and exo-) fusion DNA polymerase (e.g., as described in U.S. Patent Application No.: 60/699,937, filed July 15, 2005), cPfu DNA polymerase (Stratagene catalog # 600154), and PEF were purified using standard production protocols (no detergent present), except that non-ionic detergents were omitted from the final storage buffers. Enzymes were stored at -20°C in 50mM Tris HCl (pH 8.2), 0.1mM EDTA, 1mM DTT, and 50% glycerol. DNA polymerase storage buffers were additionally supplemented with one or more zwitterionic detergents, in percentages (v/v) ranging from 0.05% to 0.5%.

PCR reaction buffers were prepared without non-ionic detergents ("DF buffer", detergent-free buffer). For example, 1X cPfu DF-buffer contains 10mM KCl, 10mM (NH₄)₂SO₄, 20mM Tris HCl (pH 8.8), 2mM MgSO₄, and 100µg/ml BSA. Detergent-free versions of Pfu fusion buffers were also prepared, and consisted of: 40mM TrisSO₄ (pH 10), 15mM K₂SO₄, 8mM (NH₄)₂SO₄, 2mM Mg SO₄ (1X Pfu fusion DF-buffer I) or 30mM TrisSO₄ (pH 10), 40mM K₂SO₄, 1.5mM (NH₄)₂SO₄, 2mM Mg SO₄ (1X Pfu fusion DF-buffer II).

PCR reaction buffers were supplemented with 0.1 % Triton X100 (non-ionic detergent) or with one or more zwitterionic detergents or non-detergent surfactants. For example, zwitterionic detergents CHAPS, CHAPSO, 3-10, and 3-12 were obtained from AnaTrace, Inc. (Maumee, OH) and added to DF-buffers in percentages (v/v) ranging from 0.05% to 0.5%. The non-detergent surfactant, Surfynol 465, was purchased from Air Products and used in a similar fashion.

### Example 2- Using zwitterionic detergents to enhance Pfu and Pfu fusion DNA polymerase activity in endpoint PCR:

For the 0.9kb and 6kb systems, PCR reactions (50µl) were conducted with 40ng cPfu DNA polymerase in 1X cPfu DF-buffer or with 28ng or 224ng Pfu fusion DNA polymerase in 1X Pfu fusion DF-buffer I or DF-buffer II, respectively. PCR reactions also contained 2U/50µl *Pyrococcus furiosus* dUTPase (PEF), 100ng of human genomic DNA, 250µM each dNTP, and 100ng of each primer. For the 9kb system, PCR reactions (50µl) consisted of 80ng Pfu, 1.5X cPfu DF-buffer, 2U *Pyrococcus furiosus* dUTPase (PEF), 200ng of human genomic, DNA, 500µM each dNTP, and 200ng of each primer. PCR reaction buffers were supplemented with 0.1 % Triton X100 or with zwitterionic detergent(s). Reactions were cycled as described below:

| **Endpoint PCR Systems** | | |
|---|---|---|
| **Target size (gene)** | **Cycling Parameters** | **Primer sequence** |
| 0.9kb (Hα1AT) | Pfu fusion: (1 cycle) 95°C 2 min; (30 cycles) 95°C 20 sec, 58°C 20 sec, 72°C 15-30 sec.; (1 cycle) 72°C 3 min | F-5'- AGA.GCT.TGA.GGA.GAG.CAG.GAA.A GG.TGG.AAC-3' |
| | Pfu: (1 cycle) 95°C 2 min; (30 cycles) 95°C 30 sec, 58°C 30 sec, 72°C 60 sec.; (1 cycle) 72°C 10 min | R-5'- GGG.AGG.GGA.GGT.ACA.GGG.TTG.A GG.CTA.GTG-3' |
| 6kb (β globin) | Pfu fusion: (1 cycle) 95°C 2 min; (30 cycles) 95°C 20 sec, 58°C 20 sec, 72°C 90-180 sec.; (1 cycle) 72°C 3 min | F-5'- ACA.AGG.GCT.ACT.GGT.TGC.CGA.TT T.TTA.TTG-3' |
| | Pfu: (1 cycle) 95°C 2 min; (30 cycles) 95°C 30 sec, 58°C 30 sec, 72°C 12 min.; (1 cycle) 72°C 10 min | R-5'- GGG.ACT.GGC.CTC.AGA.GGA.AAC.T TC.AGG-3' |
| 9kb (β globin) | Pfu: (1 cycle) 92°C 2 min; (30 cycles) 92°C 30 sec, 58°C 30 sec, 68°C 18 min; (1 cycle) 68°C 10 min | F-5' - ACAAGGGCTACTGGTTGCCGATTTT TATTG - 3' |
| | | R-5’- GTTTGAGCAACTCTCACCATTATGG GC- 3’ |

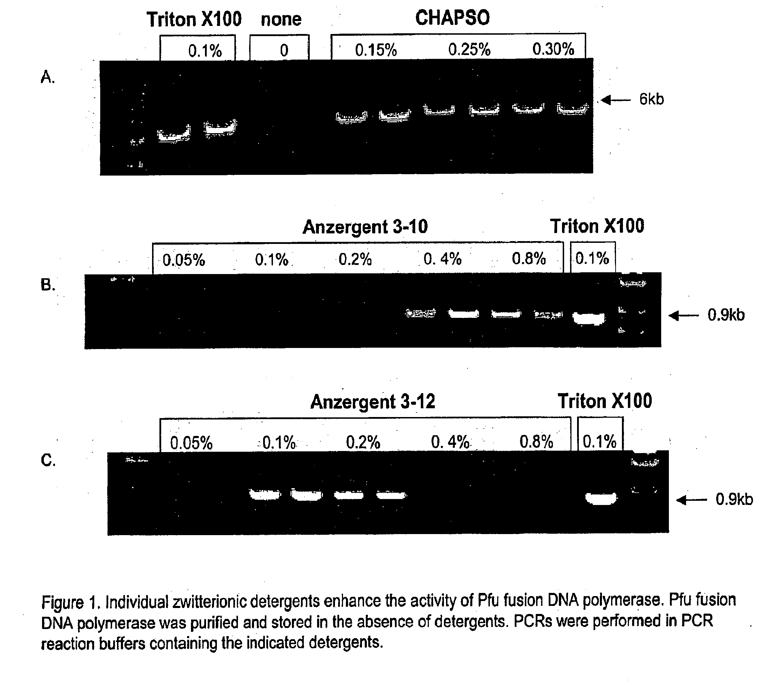

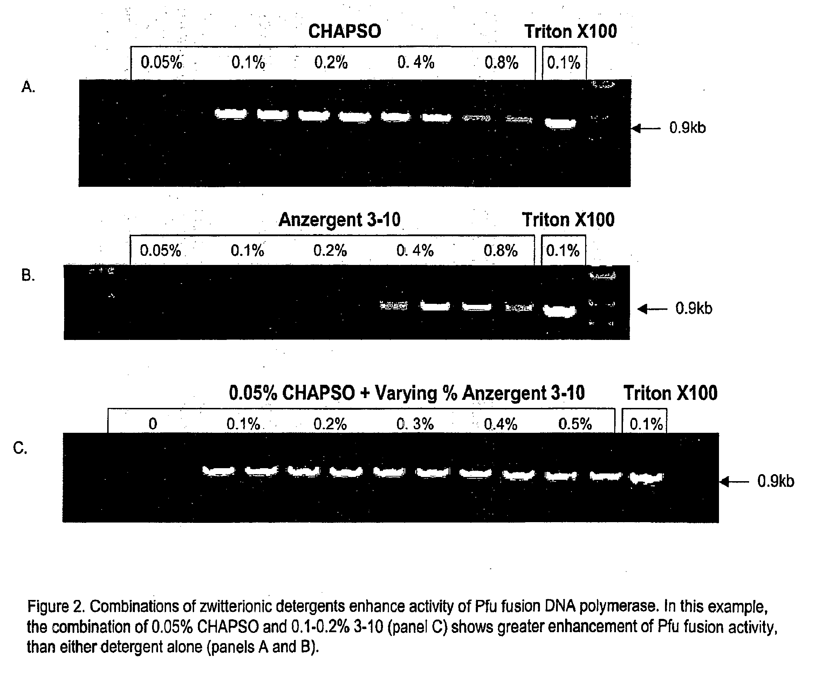

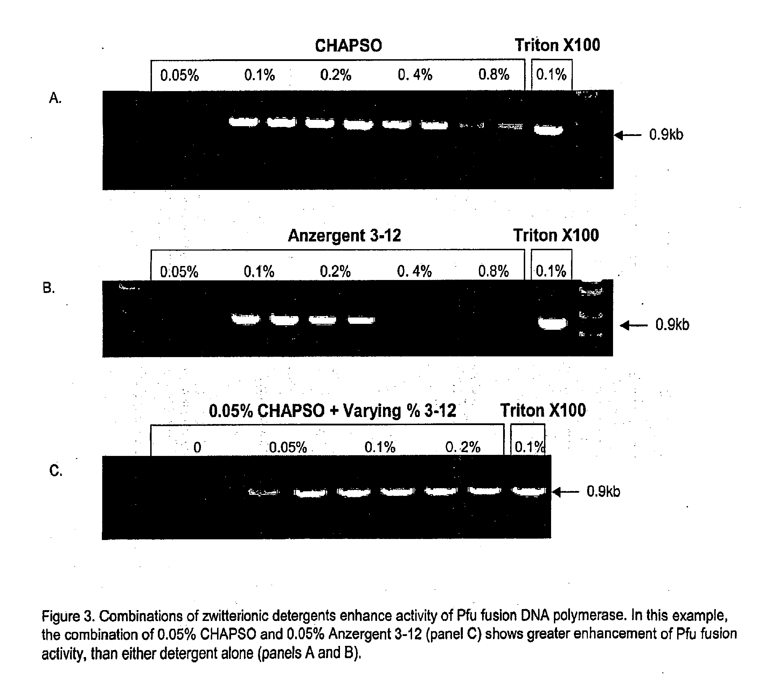

The results shown in Figures 1-8 demonstrate the enhancing and/or stabilizing activity of zwitterionic detergents. Pfu fusion DNA polymerase, purified and stored in the absence of detergent, was used to amplify genomic DNA targets in fusion PCR buffers supplemented with zwitterionic detergents (Figures 1-3). PCRs conducted in the absence of detergent failed to generate product (Figure 1, panel A). In contrast, amplifications performed in the presence of CHAPSO (0.15-0.3%), Anzergent 3-10 (0.4-0.8%), and Anzergent 3-12 (0.1-0.2%) generated high product yields (Figure 1). In some cases, combinations of zwitterionic detergents provide greater enhancement of Pfu fusion activity than individual detergents. For example, product yields are dramatically improved when suboptimal amounts of Anzergent 3-10 (0.1-0.2%; Figure 2) or 3-12 (0.05%; Figure 3) are combined with a suboptimal amount of CHAPSO (0.05%) detergent.

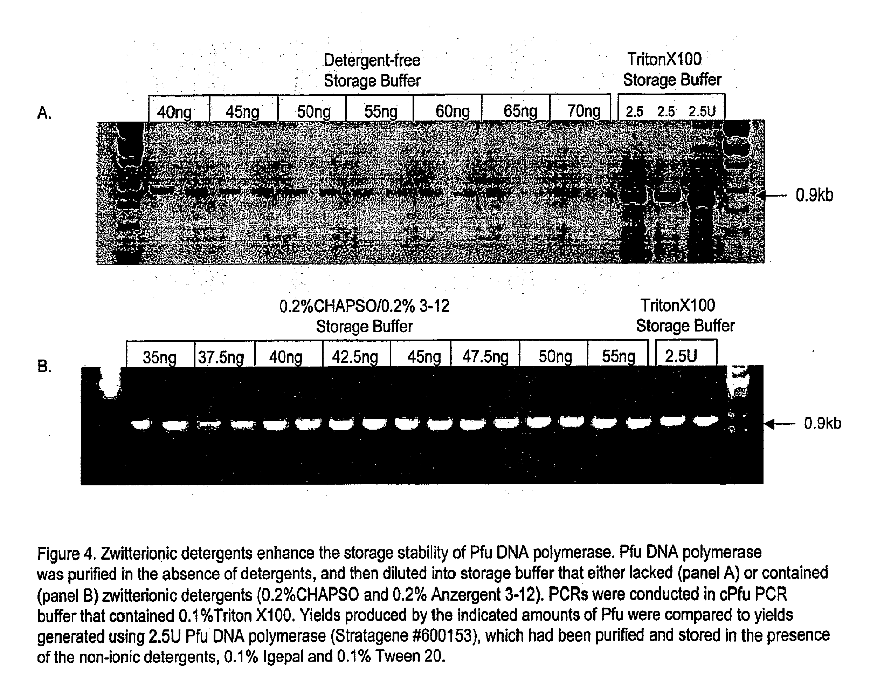

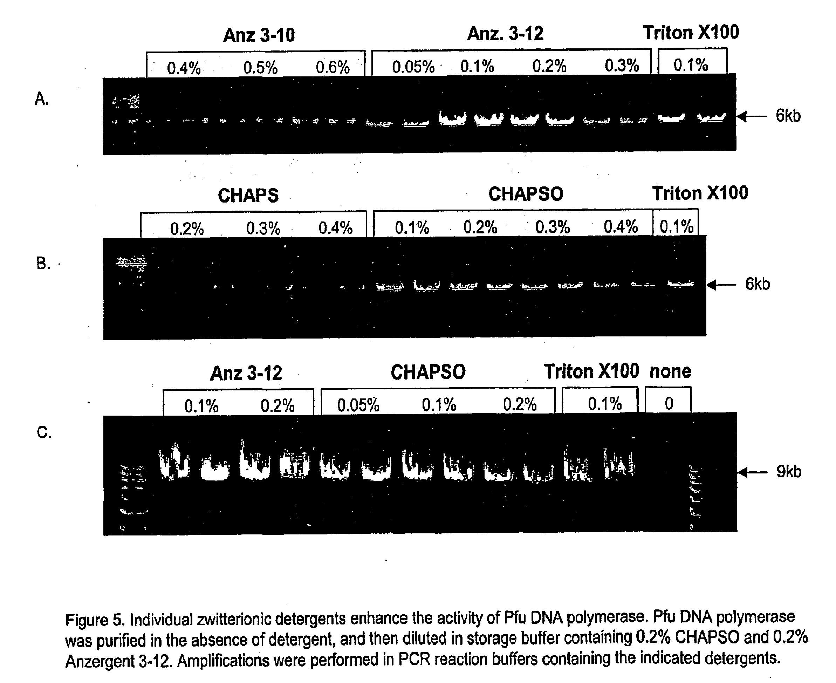

Zwitterionic detergents were also incorporated into enzyme storage buffers. For example, Pfu DNA polymerase was purified in the absence of detergent, and then diluted in storage buffers that lacked detergent (panel A, Figure 4) or contained 0.2% each of the zwitterionic detergents, CHAPSO and Anzergent 3-12 (panel B, Figure 4). When used in PCR with cloned Pfu PCR buffer (contains 0.1% Triton X100), Pfu samples prepared with zwitterionic detergents produced significantly higher yields than Pfu samples that were diluted and stored in the absence of CHAPSO and Anzergent 3-12. In addition to enhancing the storage stability of Pfu, zwitterionic detergents were also shown to increase yields when incorporated into PCR buffers (Figure 5). PCR conducted in the buffer lacking detergent failed to generate product (panel C, Figure 5). In contrast, the addition of zwitterionic detergents dramatically improved product yields, and CHAPSO (0.05-0.2%) and Anzergent 3-12 (0.1-0.2%) were found to be somewhat more effective than CHAPS and Anzergent 3-10 (Figure 5).

The detergents shown to enhance Pfu and Pfu fusion DNA polymerase activity include those listed in the following Table:

| **TABLE 1--Enhancing detergents and detergent combinations** | | | |
|---|---|---|---|
| **Effective range** | | **Optimal range** | |
| **Detergent 1** | **Detergent 2** | **Detergent 1** | **Detergent 2** |
| CHAPS (0.2-0.8%) | | CHAPS (0.2-0.4%) | |
| CHAPSO (0.1-0.8%) | | CHAPSO (0.15-0.35%) | |
| Anz.3-10 (0.4-0.8%) | | Anz.3-10 (0.4-0.8%) | |
| Anz. 3-12 (0.1-0.4%) | | Anz. 3-12 (0.1-0.2%) | |
| CHAPS (0.05, 0.1%) | Anz.3-10 (0.1-0.5%) | CHAPS (0.05, 0.1%) | Anz.3-10 (0.1-0.5%) |
| CHAPS (0.05%) | Anz.3-12 (0.05-0.5%) | CHAPS (0.05%) | Anz.3-12 (0.05-0.3%) |
| CHAPS (0.1%) | Anz.3-12 (0.05-0.5%) | CHAPS (0.1%) | Anz.3-12 (0.05-0.5%) |
| CHAPSO (0.1%) | Anz.3-10 (0.05-0.5%) | CHAPSO (0.1%) | Anz.3-10 (0.05-0.4%) |
| CHAPSO (0.05%, 0.1%) | Anz.3-12 (0.05-0.5%) | CHAPSO (0.05, 0.1%) | Anz.3-12 (0.05-0.4%) |
| CHAPS (0.05-0.5%) | CHAPSO (0.05-0.3%) | | |

| | | | |
|---|---|---|---|
| Anz., Anzergent (AnaTrace). | | | |

### Example 3- Using zwitterionic detergents to stabilize Pfu fusion enzyme

Accelerated stability studies were performed to illustrate the stabilizing effects of zwitterionic detergents. Pfu fusion DNA polymerase was purified in the absence of detergents and then diluted to 28ng/µl in storage buffer lacking detergent or storage buffers containing either conventional non-ionic detergent (0.1% Igepal/0.1% Triton X100) or various zwitterionic detergents. Protein samples were stored at -20°C or were heated at 95°C for varying lengths of time. Residual activity was assayed by amplifying a 0.9kb genomic target in PCR in fusion DF-buffer supplemented with either 0.1 % Triton X100 (A) or 0.1% CHAPSO/0.1% Anzergent 3-12.

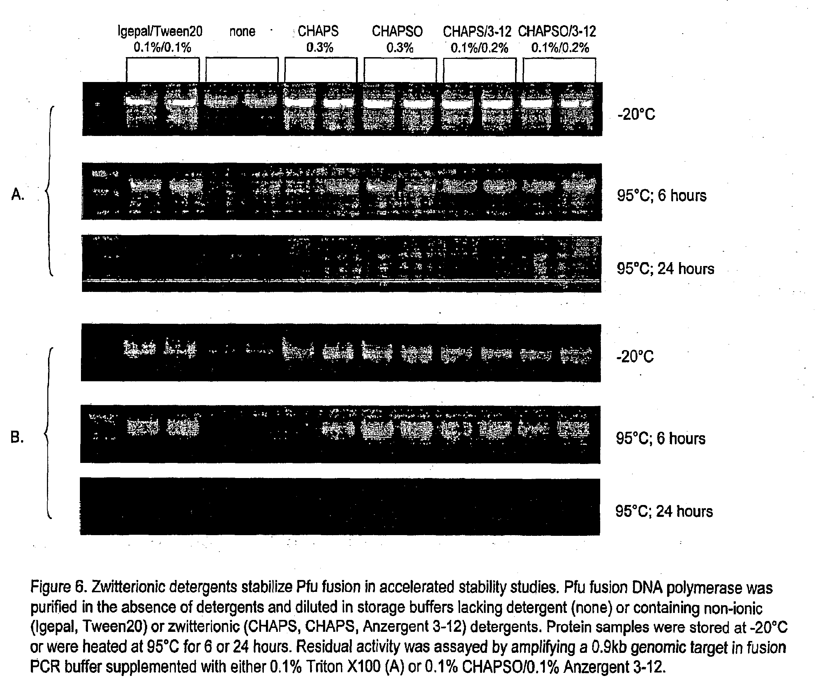

The results shown in Figure 6 indicate that zwitterionic detergents CHAPS, CHAPSO, and 3-12 enhance the stability of Pfu fusion DNA polymerase. Pfu fusion loses activity when heated at 95°C in the absence of detergent. Compared to conventional non-ionic detergents, zwitterionic detergents appear to be equally effective in protecting Pfu fusion against activity losses (95°C, 6 hours; Figure 6). After 24 hours at 95°C, protein samples stored in non-ionic and zwitterionic detergents are completely inactive.

### Example 4- Using zwitterionic detergents to enhance Pfu fusion DNA polymerase activity in QPCR

QPCR reactions contained DNA or cDNA template, varying amounts of primer (see Table 2 below), 300µM each dNTP, 4 ng/µl exo⁻Pfu fusion, 6ng/µl hot start IgG, 0.4ng/µl single-stranded DNA-binding protein, 1X Pfu fusion DF-buffer II (pH 9), 4% DMSO, and 8% glycerol. QPCR reactions were supplemented with 0.1% Triton X100 or zwitterionic detergent, and with 0.5X SYBR Green (Molecular Probes S-7567). Reactions were cycled on the MX3000P Real-Time PCR System using the following conditions: (1 cycle) 95°C 5 min; (40 cycles) 95°C 10 sec, 60°C 30 sec.

| **TABLE 2-QPCR Systems** | | | |
|---|---|---|---|
| **Target (bp)** | **Template** | **Primer Concentration** | **Primer Sequences** |
| Hu gaucher (105) | Human genomic DNA | 0.2uM each | F: CCTGAGGGCTCCCAGAGAGTGG |
| | | | R: GGTTTAGCACGACCACAACAGC |
| Hu aldolase (286) | Human genomic DNA | 0.2uM F | F: AGCCTAGCTCCAGTGCTTCTAGTA |
| | | 0.3uM R | R CTTTGGATGAGGAGCCGATATTG |
| Hu GDH (354) | Reverse-transcribed human RNA | 0.3uM each | F: GATGGATCATGGCTGACTTC |
| | | | R AGCAAGCAACTGACTGCTCT |

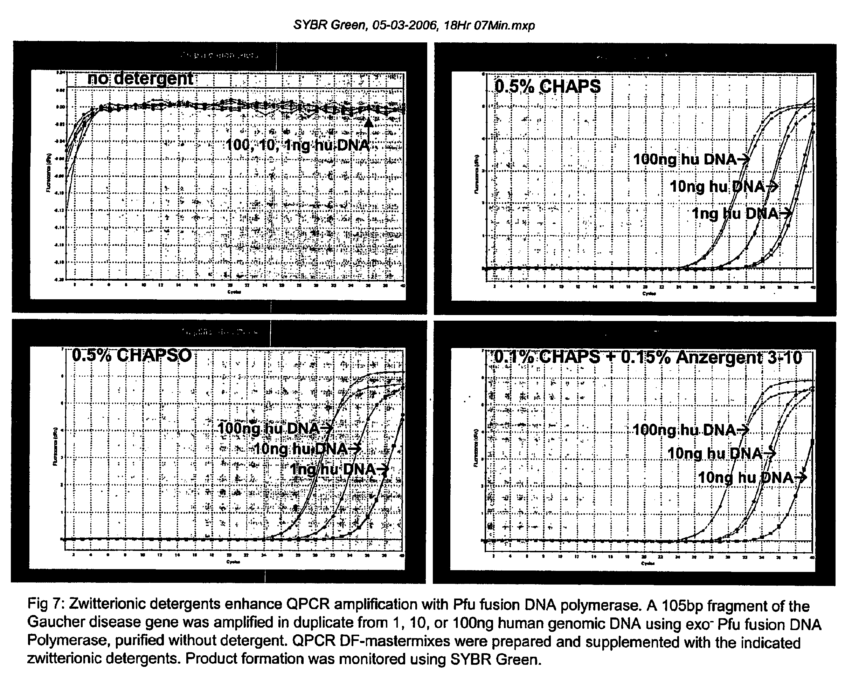

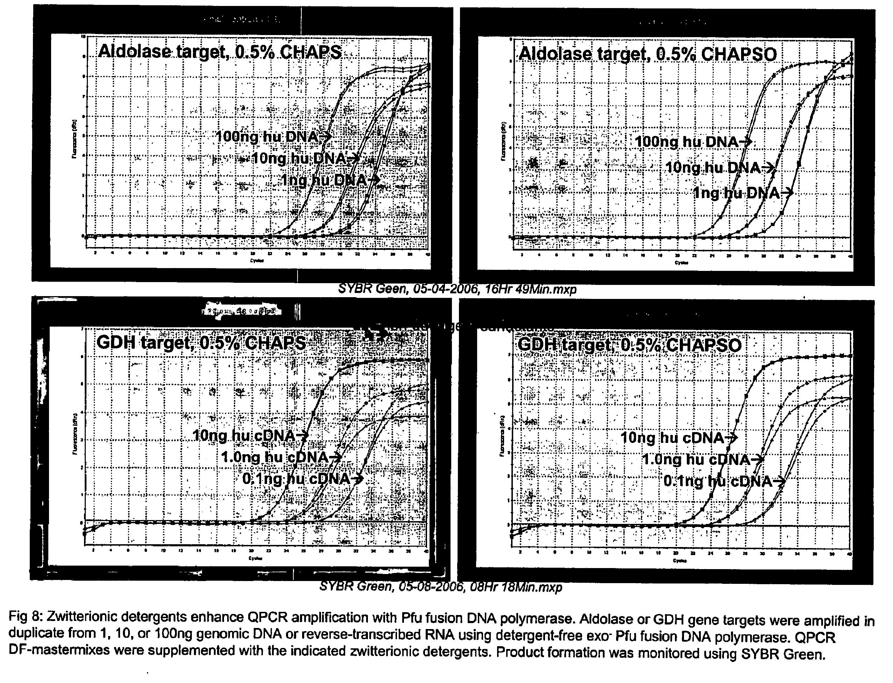

The results shown in Figures 7 and 8 demonstrate the enhancing activity of zwitterionic detergents in QPCR. Amplifications were conducted using QPCR Mastermixes (Stratagene catalog # 600581) formulated with detergent-free Pfu fusion DNA polymerase, SYBR Green, and various Zwitterionic detergents. QPCRs conducted in the absence of detergent failed to generate product (Figure 7, top left panel). In contrast, amplifications performed in the presence of 0.5% CHAPS or CHAPSO appear comparable to those conducted in the presence of conventional non-ionic detergent (0.1% Triton X100), with respect to amplification efficiency, total fluorescence, and Ct values. Combinations of zwitterionic detergents can be as effective or more effective than individual zwitterionic detergents. For example, the combination of 0.1% CHAPS and 0.15% Anzergent 3-10 is particularly effective in enhancing Pfu fusion activity in QPCR.

The following zwitterionic detergents or detergent combinations enhance QPCR amplifications conducted with Pfu fusion and SYBR Green detection:

| **TABLE 3-Enhancing detergents and detergent combinations for SYBR Green QPCR** | | | |
|---|---|---|---|
| **Effective range** | | **Optimal range** | |
| **Detergent 1** | **Detergent 2** | **Detergent 1** | **Detergent 2** |
| CHAPS (0.1-0.5%) | | CHAPS (0.5%) | |
| CHAPSO (0.1-0.75%) | | CHAPSO (0.2-0.5%) | |
| Anz.3-10 (0.1-0.3%) | | Anz.3-10 (0.2%) | |
| Anz. 3-12 (0.1-0.2%) | | Anz. 3-12 (0.15-0.2%) | |
| CHAPS (0.1%) | Anz.3-10 (0.02-0.15%) | CHAPS (0.1%) | Anz.3-10 (0.05-0.15%) |

### Example 5- Using non-detergent surfactant to enhance Pfu DNA polymerase activity in endpoint PCR

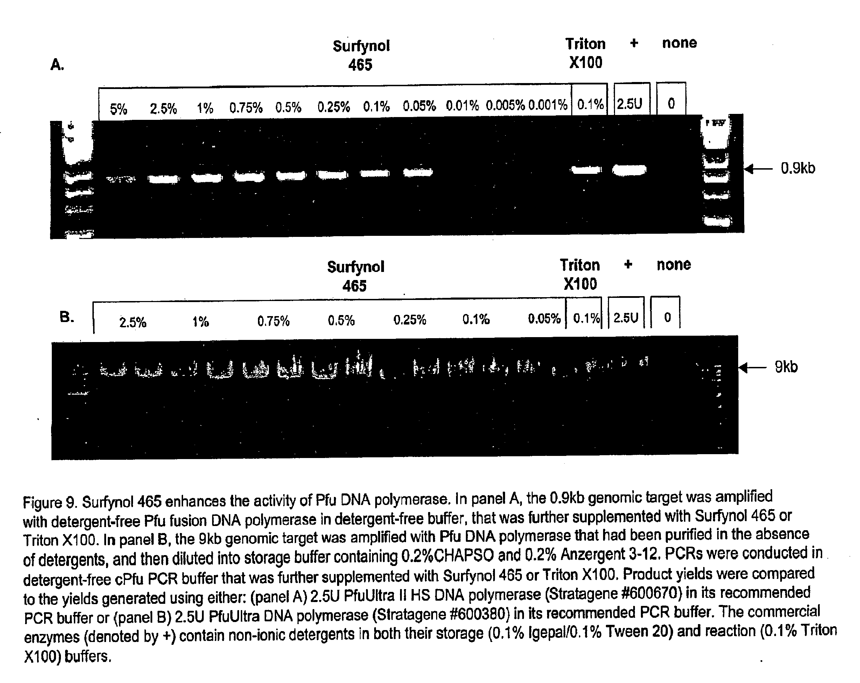

The results shown in Figure 9 demonstrate the enhancing and/or stabilizing activity of Surfynol 465 on Pfu fusion (panel A) and non-fusion (panel B) DNA polymerases. PCRs were conducted using the conditions described in Example 2. Amplifications performed in the absence of non-ionic detergents generated high product yields when PCR reaction buffers were supplemented with 0.05% to 2.5% Surfynol 465. Yields were similar to those obtained using detergent-free PCR buffers that were further supplemented with 0.1% Triton X100.

## Claims

1. A storage composition comprising a purified polymerase, a zwitterionic detergent or non-detergent surfactant, and optionally a detectable label,
wherein said zwitterionic detergent is selected from the group consisting of: a combination of n-Decyl-N,N-dimethyl-3-ammonio-1-propanesulfate (Anzergent/Zwittergent 3-10) or n-Dodecyl-N,N-dimethyl-3-ammonio-1-propanesulfonate (Anzergent/Zwittergent 3-12) with CHAPS or CHAPSO, and
wherein said non-detergent surfactant is Surfynol 465.

2. A kit comprising the storage composition of claim 1, and packaging materials therefore.

3. A method for utilizing the storage composition of claim 1 for increasing the efficiency of a polymerase comprising: forming a reaction mixture by mixing a target nucleic acid with a polymerase, a primer, an oligonucleotide probe, a detectable label, dNTPs and a zwitterionic detergent.

4. A method for preparing a purified polymerase storage composition of claim 1, the method comprising: mixing a purified polymerase, a zwitterionic detergent or non-detergent surfactant, and optionally a detectable label, in a suitable buffer.

5. The composition, kit or method of any one of claims 1-4, wherein the polymerase is thermostable.

## Patentansprüche

1. Eine Zusammensetzung für die Lagerung, die eine gereinigte Polymerase, ein zwitterionisches Detergens oder ein Detergens-freies oberflächenaktives Mittel, und gegebenenfalls einen nachweisbaren Marker umfasst,
wobei das zwitterionische Detergens ausgewählt ist aus der Gruppe bestehend aus: einer Kombination von n-Decyl-N,N-dimethyl-3-ammonium-1-propansulfat (AnzergentlZwittergent 3-10) oder n-Dodecyl-N,N-dimethyl-3-ammonium-1-propansulfonat (Anzergent/Zwittergent 3-12) mit CHAPS oder CHAPSO, und
wobei das Detergens-freie oberflächenaktive Mittel Surfynol 465 ist.

2. Ein Kit umfassend die Zusammensetzung für die Lagerung nach Anspruch 1 und Verpackungsmaterialien dafür.

3. Ein Verfahren zur Nutzung der Zusammensetzung für die Lagerung nach Anspruch 1 für das Steigern der Effizienz einer Polymerase umfassend: Bilden einer Reaktionsmischung durch Mischen einer Zielnukleinsäure mit einer Polymerase, einem Primer, einer Oligonukleotidsonde, einem nachweisbaren Marker, dNTPs und einem zwitterionischen Detergens.

4. Ein Verfahren zur Herstellung einer Zusammensetzung für die Lagerung einer gereinigten Polymerase nach Anspruch 1, wobei das Verfahren umfasst: Mischen einer gereinigten Polymerase, eines zwitterionischen Detergens oder eines Detergens-freien oberflächenaktiven Mittels, und gegebenenfalls einem nachweisbaren Marker, in einem geeigneten Puffer.

5. Die Zusammensetzung, das Kit oder die Verfahren nach einem der Ansprüche 1-4, wobei die Polymerase thermostabil ist.

## Revendications

1. Composition de stockage comprenant un polymèrase purifié, un détergent zwittérionique ou le tensio-actif non détergent, et éventuellement une étiquette détectable,
où ledit détergent zwittérionique est sélectionné parmi le groupe consistant en: une combinaison de n-Décyle-N,N-diméthyle-3-ammonio-1-propanesulfate (Anzergent/ Zwittergent 3-10) ou de n-Dodécyle-N,N-diméthyle-3-ammonio-1-propanesulfonate (Anzergent/Zwittergent 3-12) avec CHAPS ou CHAPSO, et
où ledit tensio-actif non détergent est le Surfynol 465.

2. Kit comprenant la composition de stockage selon la revendication 1, et matériaux de conditionnement prévus à cet effet.

3. Procédé d'utilisation de la composition de stockage selon la revendication 1 permettant d'augmenter l'efficacité d'un polymérase comprenant: la formation d'un mélange réactionnel consistant à mélanger un acides nucléique cible avec un polymérase, un initiateur, une sonde oligonucléotide, une étiquette détectable, des dNTP et un détergent zwittérionique.

4. Procédé de préparation d'une composition de stockage de polymérase purifié selon la revendication 1, le procédé comprenant: le mélange d'un polymérase purifié, d'un détergent zwittérionique ou d'un tensio-actif non détergent, et éventuellement, une étiquette détectable, dans un tampon bien choisi.

5. Composition, kit or procédé selon l'une quelconque des revendications 1-4, où le polymérase est thermostable.
